Europäisches Patentamt

European Patent Office

Office européen des brevets

(11) Veröffentlichungsnummer: **0 257 352 B1**

## EUROPÄISCHE PATENTSCHRIFT

(45) Veröffentlichungstag der Patentschrift: **10.06.92**

(21) Anmeldenummer: **87111139.9**

(22) Anmeldetag: **01.08.87**

(51) Int. Cl.5: **G01N 33/543**, G01N 33/536, G01N 33/74, //G01N33/82, G01N33/60,G01N33/577

(54) Verfahren und Testkit zur Bestimmung freier Wirkstoffe in biologischen Flüssigkeiten.

(30) Priorität: **05.08.86 DE 3626468**

(43) Veröffentlichungstag der Anmeldung:
**02.03.88 Patentblatt 88/09**

(45) Bekanntmachung des Hinweises auf die Patenterteilung:
**10.06.92 Patentblatt 92/24**

(84) Benannte Vertragsstaaten:
**AT BE CH DE ES FR GB IT LI NL SE**

(56) Entgegenhaltungen:
**EP-A- 0 026 103     EP-A- 0 066 285**
**EP-A- 0 089 806     EP-A- 0 106 615**
**EP-A- 0 155 104     WO-A-81/01414**
**WO-A-85/00226       GB-A- 2 030 290**
**US-A- 3 928 553**

**CHEMICAL ABSTRACTS, Band 76, Nr.5, 31 Januar 1972, Columbus, OH (US); H.GHARIB et al., Seite 167, Nr.22720f**

**CLINICAL CHEMISTRY, Band 30, Nr.7, Juli 1984, Winston-Salem, NC (US); J.A.HINDS et al., Seiten 1174-1178**

(73) Patentinhaber: **HOECHST AKTIENGESELL-SCHAFT**
**Postfach 80 03 20**
**W-6230 Frankfurt am Main 80(DE)**

(72) Erfinder: **Simons, Guido, Dr.**
**Talstr. 24**
**W-6507 Ingelheim am Rhein(DE)**
Erfinder: **Strecker, Helmut, Dr.**

**verstorben(DE)**
Erfinder: **Molz, Peter, Dr.**
**Kaiser Wilhelm-Ring 44**
**W-6500 Mainz(DE)**
Erfinder: **Schnorr, Gerd, Dr.**
**Auf dem Lattigkopf 23**
**W-6368 Bad Vilbel(DE)**
Erfinder: **Skrzipczyk, Heinz Jürgen, Dr.**
**Am Schellberg 16**
**W-6232 Bad Soden am Taunus(DE)**
Erfinder: **Wissmann, Hans, Dr.**
**Falkenstr. 12**
**W-6232 Bad Soden(DE)**

Rank Xerox (UK) Business Services

CLINICAL CHEMISTRY, Band 29, Nr.10, Oktober 1983, Washington, DC (US); N.P.KUBASIK et al., Seiten 1781-1786

**Beschreibung**

Gegenstand der Erfindung ist ein Verfahren und ein Testkit zur Bestimmung der Konzentration des freien Anteils eines in einer biologischen Flüssigkeit vorhandenen Wirkstoffes in Gegenwart von natürlichen Bindemitteln wie Proteinen, wobei der freie und der gebundene Anteil des Wirkstoffes miteinander im Gleichgewicht stehen.

Es ist für die meisten physiologisch aktiven Substanzen bekannt, daß sie in biologischen Flüssigkeiten wie dem Blut teils in freier Form, teils aber auch an Proteine wie Globuline oder Albumine gebunden vorliegen. Dabei stehen die freie und die gebundene Form des Wirkstoffes miteinander im Gleichgewicht.

Es wird derzeit angenommen, daß nur der Anteil des Wirkstoffes physiologische Wirkungen entfaltet, der nicht an Proteine gebunden ist. Denn durch die Bindung an Proteine verliert der Wirkstoff die Fähigkeit, mit seinem spezifischen Rezeptor zu reagieren, was Voraussetzung für seine Wirksamkeit ist. Es konnte auch schon gezeigt werden, daß bestimmte Arzneimittel ihre Wirkung verlieren, wenn sie an Albumine im Serum gebunden werden, während sich ihre Wirkung durch Zugabe von Substanzen erhöht, die sie aus der Albuminbindung verdrängen. Es sind deshalb auch schon eine Reihe von diagnostischen Verfahren entwickelt worden, mit denen nur der nicht an Proteine gebundene Anteil eines Wirkstoffes bestimmt werden kann.

So ist aus dem Europäischen Patent 26 103 ein Verfahren zur Bestimmung der Konzentration des freien Anteils eines in einer biologischen Flüssigkeit vorhanden Wirkstoffes bekannt, bei dem die zu untersuchende Probe mit einem markierten Derivat des Wirkstoffes und mit einem spezifischen Bindemittel für den Wirkstoff vermischt werden, und nach der Umsetzung dieser Reagenzien die Menge des markierten Derivats des Wirkstoffes gemessen wird, welche an das spezifische Bindemittel gebunden oder nicht gebunden ist. Hieraus läßt sich dann die Konzentration des freien Wirkstoffes in der biologischen Flüssigkeit berechnen. Mit diesem Verfahren lassen sich zuverlässige Meßergebnisse jedoch nur dann erzielen, wenn das markierte Derivat des Wirkstoffes so ausgewählt ist, daß es sich praktisch ausschließlich mit dem spezifischen Bindemittel verbindet, nicht jedoch mit den natürlichen Bindungsproteinen, die in der biologischen Flüssigkeit anwesend sind. Dieses Erfordernis läßt sich in der Praxis in vielen Fällen aber nicht befriedigend erfüllen.

Deshalb wurde in der Europäischen Patentanmeldung 155 104 auch schon vorgeschlagen, der Probe der zu untersuchenden biologischen Flüssigkeit außer dem markierten Derivat des Wirkstoffes und dem spezifischen Bindemittel eine weitere Substanz zuzusetzen, die die Bindung des markierten Derivats des Wirkstoffes an die natürlichen Proteine blockieren soll, in dem sie selbst die Bindungsstellen des Proteins besetzt. Dieses Verfahren wird auch in der Deutschen Patentschrift 34 15 818 beschrieben.

Auch die internationale Patentanmeldung WO 85/00 226 versucht das Problem der Bindung des markierten Wirkstoffderivats an die natürlichen Proteine und die dadurch verursachten Meßfehler zu lösen. Dort wird vorgeschlagen, der den freien Wirkstoff enthaltenden biologischen Flüssigkeit ein spezifisches Bindemittel für den freien Wirkstoff, ein markiertes Derivat des Wirkstoffes und außerdem noch ein spezielles Bindemittel für das markierte Wirkstoffderivat zuzusetzen. Das markierte Wirkstoffderivat (Tracer) wird dann sowohl mit dem spezifischen Bindemittel für den Wirkstoff selbst als auch mit dem Bindemittel für das Wirkstoffderivat reagieren, nicht aber mit den Bindungsproteinen, weil diese eine viel geringere Affinität zum Tracer haben als das spezifische Bindemittel für den Tracer.

Ein Verfahren zur Bestimmung der Gesamt-Konzentration eines Analyten, d. h. der Summe aus freiem und gebundenem Anteil, in einem nicht-homogenen Enzymimmunoassay wird in der WO 81/01414 beschrieben. Zur Verdrängung des enzymmarkierten Analyten (Tracer) vom Antikörper sind allerdings hohe Analytkonzentrationen notwendig. Für die Bestimmung des freien Analytgehaltes ist dieses Verfahren nicht geeignet.

Die vorstehend genannten Veröffentlichungen zeigen, daß die unerwünschte Bindung des Tracers an natürliche Proteine zu einer ernstlichen Beeinträchtigung der Meßgenauigkeit bei der Bestimmung des freien Anteils eines Wirkstoffes in einer biologischen Flüssigkeit führt und daß ein Bedürfnis besteht, dieses Problem auf möglichst einfache Weise zu lösen. Die vorliegende Erfindung stellt sich deshalb die Aufgabe, dieses Problem ohne die zusätzliche Verwendung eines die Bindungsstelle der natürlichen Proteine blockierenden Mittels und auch ohne den Zusatz eines spezifischen Bindemittels für den Tracer zu lösen.

Es wurde nun gefunden, daß sich die Konzentration des freien Anteils eines in einer biologischen Flüssigkeit vorhandenen Wirkstoffes in Gegenwart von natürlichen Bindemitteln, wobei der freie und der gebundene Anteil des Wirkstoffes miteinander im Gleichgewicht stehen, und die Menge des unmarkierten Antikörpers und/oder seiner Affinität zum Wirkstoff so gering sind, daß sie das Gleichgewicht zwischen freiem und gebundenem Anteil des Wirkstoffs nicht wesentlich beeinflussen, bestimmen läßt, indem man

a) eine Probe der Flüssigkeit mit einem unmarkierten Antikörper in Berührung bringt;

b) die Probe von unmarkierten Antikörper abtrennt;

c) den unmarkierten Antikörper mit einer markierten, mit ihm kreuzreagierenden Substanz (Tracer) inkubiert;

d) den Anteil des Tracers mißt, der an den Antikörper gebunden oder nichtgebunden ist und daraus die Konzentration des freien Anteils des Wirkstoffes errechnet,

und wobei der Tracer eine vom zu bestimmenden Wirkstoff abweichende Molekülstruktur aufweist, die nicht durch die Markierung hervorgerufen ist, und eine erheblich höhere oder eine erheblich geringere Affinität zum Antikörper hat als der Wirkstoff selbst.

Besonders geeignet ist dieses Verfahren für die Bestimmung des freien Anteils von Thyroxin, Trijodthyronin und von Steroid-Hormonen in biologischen Flüssigkeiten.

Das ertindungsgemäße Verfahren unterscheidet sich von den in den vorstehend genannten Veröffentlichungen beschriebenen Bestimmungsverfahren zunächst einmal dadurch, daß die Probe der zu untersuchenden biologischen Flüssigkeit nach der Reaktion mit dem unmarkierten Antikörper abgetrennt wird. Damit werden die störenden natürlichen Bindungsproteine entfernt und können den weiteren Verlauf des Bestimmungsverfahrens nicht mehr stören und auch keine Meßungenauigkeiten verursachen.

Ein derartiges zweistufiges Verfahren ist an sich aus der Deutschen Offenlegungsschrift 29 36 307 bekannt. Dort wird ebenfalls eine Methode zur Bestimmung des freien Anteils eines Wirkstoffes beschrieben, in dem man die zu untersuchenden Flüssigkeitsproben mit einem unmarkierten Rezeptor in Kontakt bringt, damit an ihn der freie Wirkstoff gebunden wird. Dann wird die Flüssigkeitsprobe entfernt und der unmarkierte Rezeptor mit einem markierten Wirkstoffderivat inkubiert und anschließend die am Rezeptor gebundene Menge des markierten Reagenz gemessen. Dieses Verfahren hat jedoch den großen Nachteil, daß in der ersten Stufe so große Mengen an unmarkiertem Rezeptor verwendet werden, daß tatsächlich der gesamte Wirkstoff gebunden wird. Da der freie Wirkstoff und der an Proteine gebundene Wirkstoff aber miteinander im Gleichgewicht stehen, wird durch die Bindung des freien Wirkstoffes an den Rezeptor das Gleichgewicht gestört und zusätzlich Wirkstoff aus seiner an Proteinen gebunden Form freigesetzt. Dadurch werden nach dieser Methode zu hohe Konzentrationen an freiem Wirkstoff gemessen.

Es ist deshalb ein Merkmal der vorliegenden Erfindung, daß der unmarkierte Antikörper in einer so kleinen Menge eingesetzt wird, daß er das Gleichgewicht zwischen freiem und an Protein gebundenem Wirkstoff nicht merklich beeinflussen kann. Aus dem gleichen Grund darf die Affinität des unmarkierten Antikörpers zum Wirkstoff nur gering sein. Es soll weder der gesamte freie Wirkstoff am unmarkierten Antikörper gebunden werden, noch sollen sämtliche freien Bindungsstellen des unmarkierten Antikörpers besetzt werden.

Ein für die Bestimmungsmethode geeigneter unmarkierter Antikörper wird zweckmäßigerweise aus der Gruppe der polyklonalen oder monoklonalen Antikörper ausgewählt und seine Affinität zum Wirkstoff in einigen wenigen routinemäßigen Vorversuchen ermittelt. Nach Abtrennung der zu untersuchenden Flüssigkeitsprobe vom unmarkierten Antikörper wird dieser anschließend mit einer markierten, mit ihm kreuzreagierenden Substanz (Tracer) inkubiert. Der Tracer wird entweder mit einem radioaktiven Atom wie Jod-125, oder einer fluoreszierenden oder chemilumineszenten Verbindung markiert. Ebenso ist auch die Markierung mit einem Enzym oder einem Lichtchromophor möglich.

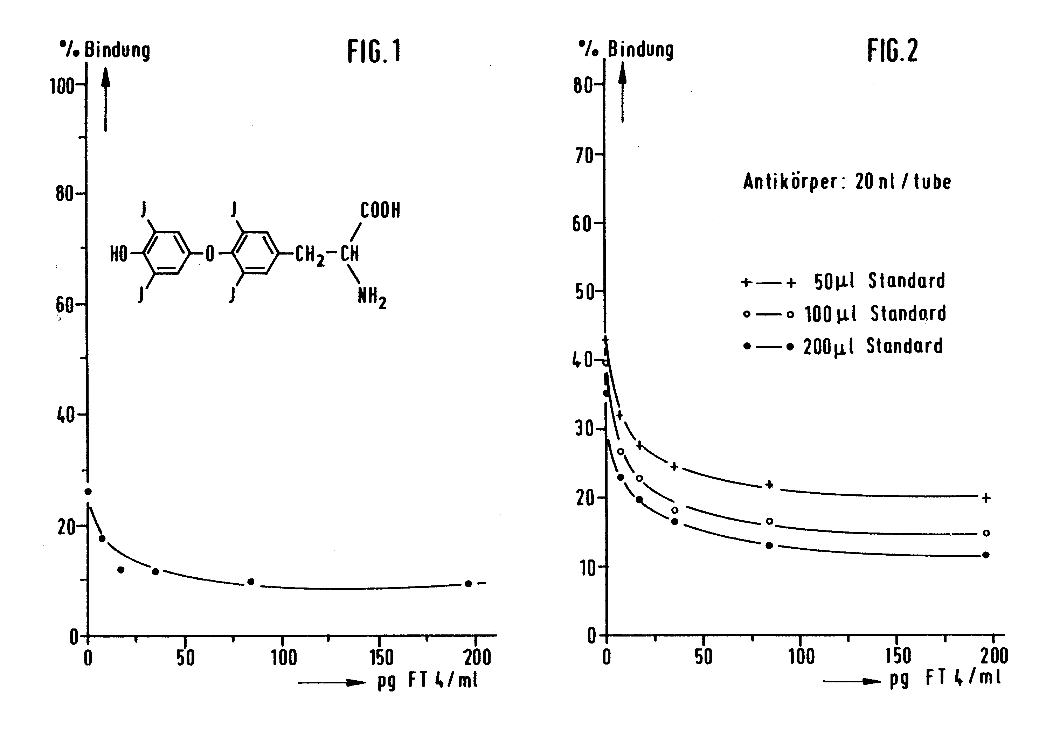

Wichtig ist, daß der Tracer eine vom zu bestimmenden Wirkstoff abweichende Molekülstruktur aufweist. Würde man beispielsweise bei der Bestimmung von Thyroxin nach dem erfindungsgemäßen Verfahren als Tracer ein mit Jod-125 markiertes Thyroxin einsetzen, dann erhielte man die in Figur 1 gezeichnete sehr flache Standardkurve, aus der eindeutige Meßergebnisse nicht mehr abgelesen werden können. Die Kurve zeigt die Meßwerte, die nach einstündiger Inkubation einer Serumprobe bei steigendem Gehalt von freiem Thyroxin mit einem Thyroxin-Antikörper, Abtrennen der Serumprobe und einer zweiten Inkubationszeit von 1 Stunde in Gegenwart von Jod-125-Thyroxin als Tracer erhalten wurden.

Wie Figur 2 zeigt, läßt sich dieser unbefriedigende Verlauf der Standardkurve auch nicht durch Variation des Probenvolumens bei sonst gleichen Meßbedingungen verbessern. Die mit 50 μl, 100 μl und 200 μl aufgenommenen Standardkurven zeigen in den für die Messung wichtigen Bereichen einen zu flachen Verlauf.

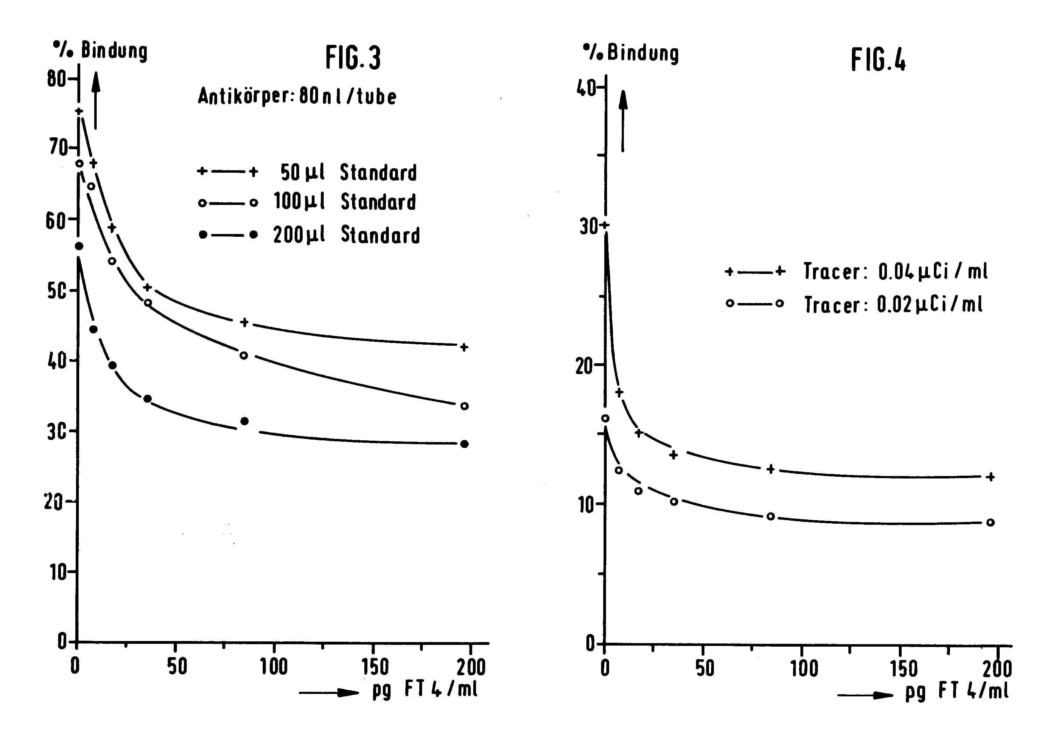

Während bei den in Figur 2 gezeigten Messungen eine Antikörperkonzentration von 20 nl auf der inneren Oberfläche eines Teströhrchens aufgebracht wurden, zeigt Figur 3 den gleichen Versuch bei Erhöhung der Antikörpermenge auf 80 nl pro Untersuchung. Auch das führt zu keinem steileren Kurvenverlauf im für die Messung wichtigen Bereich.

Figur 4 zeigt die Meßwerte, die bei einer Wiederholung der in Figur 1 gezeigten Messungen unter Verwendung von Tracern mit unterschiedlicher Radioaktivität erhalten wurden. Die Traceraktivitäten betrugen 0,04 μCi und 0,02 μCi. Die Aussagekraft der Standardkurven wurde dadurch nicht verbessert.

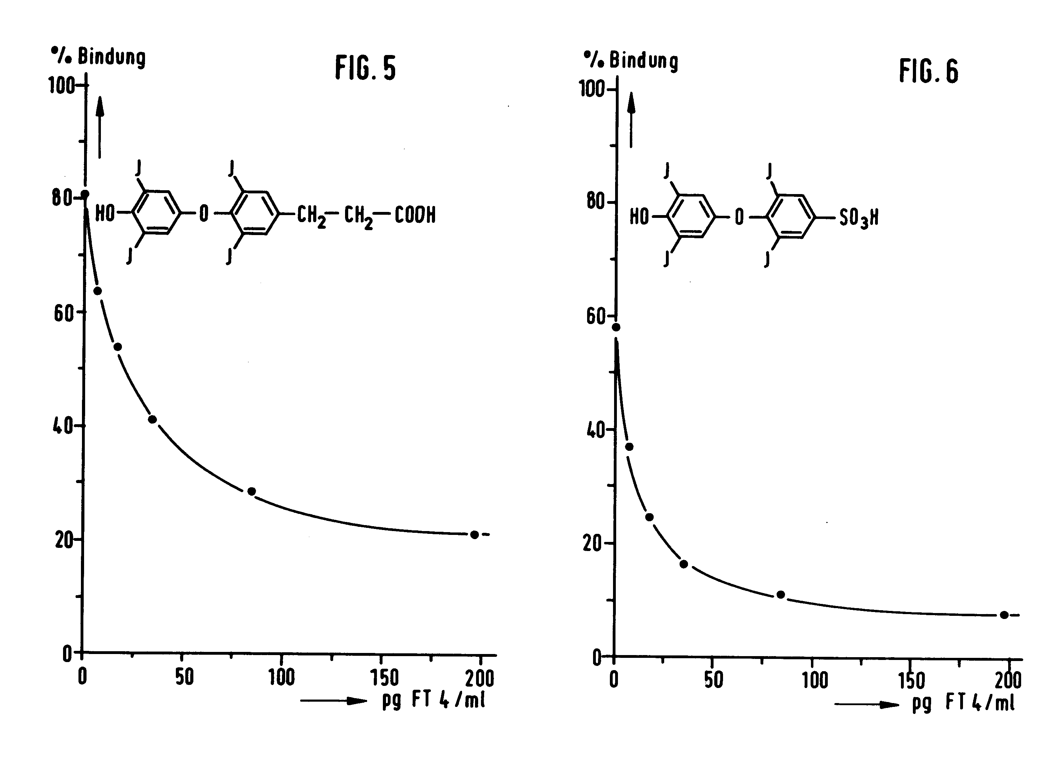

Setzt man jedoch für die Bestimmung von Thyroxin nach dem erfindungsgemäßen Verfahren ein Derivat des Thyroxins ein, das an der Amino-, an der Carboxygruppe oder an einer anderen Stelle des Moleküls modifiziert wurde, dann erhält man Standardkurven, wie sie in den Figuren 5 bis 8 gezeigt sind, bei denen jeder an den Antikörper gebundenen Tracermenge eine bestimmte Thyroxinkonzentration eindeutig zugeordnet werden kann. Geeignete Tracer für die Bestimmung von Thyroxin und Trijodthyronin, welche nach dem erfindungsgemäßen Verfahren eingesetzt werden können, sind in der Deutschen Patentanmeldung P 36 00 365.4 beschrieben.

Aufgrund seiner geänderten chemischen Struktur hat der Tracer eine höhere oder geringere Affinität zum Antikörper als der Wirkstoff selbst. Im allgemeinen ist es vorteilhaft, einen Tracer auszuwählen, der eine größere Affinität zum Antikörper hat als der Wirkstoff selbst. Ist jedoch die Konzentration des zu bestimmenden freien Wirkstoffes sehr niedrig, dann ist ein Tracer mit einer erheblich geringeren Affinität zum Antikörper als der Wirkstoff vorzuziehen. Denn durch den in sehr niedriger Konzentration vorliegenden Wirkstoff werden nur wenige Bindungsstellen des Antikörpers besetzt, was kaum zu erkennen wäre, wenn anschließend ein Tracer mit hoher Affinität zugegeben würde. Es könnte dann der Eindruck entstehen, daß überhaupt kein freier Wirkstoff vorhanden ist. Demgegenüber läßt sich ein einwandfreies Meßergebnis dann immer noch erzielen, wenn ein Tracer mit geringerer Affinität verwendet wird.

Grundsätzlich sind als Tracer alle Substanzen einsetzbar, die eine andere Affinität als der zu bestimmende Wirkstoff zu dem Antikörper haben, aber mit ihm um die freien Bindungsstellen des Antikörpers in einer Kreuzreaktion konkurrieren. Deshalb können als Antikörper auch antiidiotype Antikörper eingesetzt werden, die sich an den im erfindungsgemäßen Verfahren eingesetzten Antikörper binden. Ein auf diesem Prinzip aufgebauter Assay ist in der Europäischen Patentanmeldung 106.615 beschrieben.

Für das erfindungsgemäße zweistufige Verfahren ist es somit kennzeichnend, daß sich der zu bestimmende Wirkstoff und der Tracer in ihrer Affinität gegenüber dem Antikörper unterscheiden. Dagegen ist es für die einstufigen Bestimmungsverfahren, wie sie beispielsweise aus der Europäischen Patentschrift 26 103 bekannt sind, kennzeichnend, daß der zu bestimmende Wirkstoff und der Tracer unterschiedliche Affinitäten gegenüber den Bindungsproteinen aufweisen.

Damit wird eine Bestimmungsmethode zur Verfügung gestellt, die die Fehlermöglichkeiten vermeidet, die allen einstufigen Verfahren zur Ermittlung des freien Anteils eines Wirkstoffes in einer biologischen Flüssigkeit auf Grund der Anwesenheit von natürlichen Bindungsproteinen anhaften. Sie zeichnet sich deshalb durch sehr präzise Meßwerte aus und kommt mit nur zwei Reagenzien aus.

Ein zur Durchführung des erfindungsgemäßen Bestimmungsverfahrens geeigneter Testkit enthält somit einen mit dem zu bestimmenden Wirkstoff reagierenden unmarkierten Antikörper, dessen Menge und/oder Affinität zum Wirkstoff so gering ist, daß er das Gleichgewicht zwischen freiem und gebundenem Anteil des Wirkstoffes nicht wesentlich beeinflußt und außerdem einen Tracer, der eine wesentlich höhere oder geringere Affinität zum Antikörper hat als der Wirkstoff selbst. Ein derartiger Testkit kann so zusammengestellt werden, daß er die Bestimmung des freien Anteils eines beliebigen Hormons, eines Steroides, eines Arzneimittels, eines Arzneimittelmetaboliten, eines Polypeptids, eines Vitamins, eines Tumorantigens, eines Toxins oder eines Alkaloids erlaubt. Besonders bevorzugt ist die Bestimmung des freien Anteils des Thyroxins, Trijodthyronins und eines Steroidhormons.

Als besonders geeignete Tracerverbindungen für den quantitativen Nachweis von Thyroxin in biologischen Flüssigkeiten nach dem erfindungsgemäßen Verfahren haben sich die folgenden Verbindungen erwiesen:

(1) 3,3′,5,5′-Tetrajodthyroessigsäure (Fig. 5)

(2) 3,5-Dijod-4-(3,5-dijod-4-oxyphenyl)-benzolsufonsäure (Fig. 6)

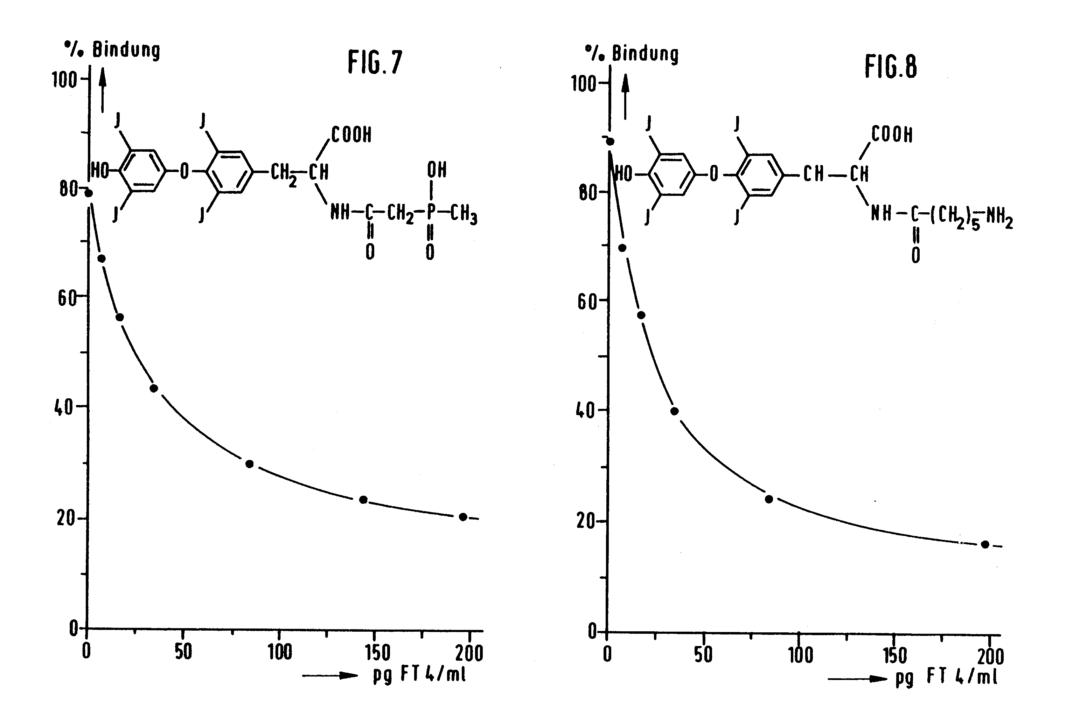

(3) N-(Methylphosphinoacetyl)-3,3′,5,5′-tetrajodthyrosin (Fig. 7)

(4) N-($\epsilon$-Aminocaproyl)-3,3′,5,5′-tetrajodthyrosin (Fig. 8)

Allgemeine Arbeitsvorschrift zur Bestimmung von freiem Thyroxin nach dem erfindungsgemäßen Verfahren

In Röhrchen, die mit T4-Antikörper beschichtet sind, (20 ng Antikörper pro Röhrchen) werden 200 µl einer Standardreihe (Humanseren mit steigendem Gehalt an freiem Thyroxin) und 1000 µl Puffer eine halbe Stunde unter Schütteln in Kontakt gebracht.

Nach dem Ausschütten der Reaktionslösung werden 1000 µl Tracer (Aktivität ca. 60.000 Impulse pro Minute) in das vorinkubierte Röhrchen gegeben. Es wird eine Stunde inkubiert, der nicht gebundene Tracer abgetrennt und in einem γ-Zähler gemessen.

Die Ergebnisse sind in Fig. 5 bis Fig. 8 dargestellt.

**Patentansprüche**

1. Verfahren zur Bestimmung der Konzentration des freien Anteils eines in einer biologischen Flüssigkeit vorhandenen Wirkstoffs in Gegenwart von natürlichen Bindemitteln, wobei der freie und der gebundene Anteil des Wirkstoffes miteinander im Gleichgewicht stehen und die Menge des unmarkierten Antikörpers und/oder seiner Affinität zum Wirkstoff so gering sind, daß sie das Gleichgewicht zwischen freiem und gebundenem Anteil des Wirkstoffes nicht wesentlich beeinflussen, indem man

   a) eine Probe der Flüssigkeit mit einem unmarkierten Antikörper in Berührung bringt;
   b) die Probe von unmarkierten Antikörpern abtrennt;
   c) den unmarkierten Antikörper mit einer markierten, mit ihm kreuzreagierenden Substanz (Tracer) inkubiert;
   d) den Anteil des Tracers mißt, der an den Antikörper gebunden oder nichtgebunden ist und daraus die Konzentration des freien Anteil des Wirkstoffes errechnet,

   dadurch gekennzeichnet, daß der Tracer eine vom zu bestimmenden Wirkstoff abweichende Molekülstruktur aufweist, die nicht durch die Markierung hervorgerufen ist, und eine erheblich höhere oder eine erheblich geringere Affinität zum Antikörper hat als der Wirkstoff selbst.

2. Verfahren nach Anspruch 1, dadurch gekennzeichnet, daß der freie Anteil des zu bestimmenden Wirkstoffes Thyroxin, Trijodthyronin oder ein Steroid-Hormon ist.

3. Verfahren nach Anspruch 1, dadurch gekennzeichnet, daß der unmarkierte Antikörper ein polyklonaler oder monoklonaler Antikörper ist.

4. Verfahren nach Anspruch 1, dadurch gekennzeichnet, daß der Tracer mit einem radioaktiven Atom, einer fluoreszierenden oder chemilumineszenten Gruppe, einem Enzym oder einem Lichtchromophor markiert ist.

5. Verfahren nach Anspruch 4, dadurch gekennzeichnet, daß man zur Bestimmung des freien Anteils von Thyroxin oder Trijodthyronin als Tracer ein Derivat des Thyroxins oder des Trijodthyronins einsetzt, welches an der Amino-, an der Carboxygruppe oder an einer anderen Stelle des Moleküls modifiziert wurde.

6. Verfahren nach Anspruch 5, dadurch gekennzeichnet, daß die Markierung des Tracers durch radioaktives Jod-125 erfolgt.

7. Testkit zur Bestimmung der Konzentration des freien Anteils eines in einer biologischen Flüssigkeit vorhandenen Wirkstoffes gemäß Anspruch 1, dadurch gekennzeichnet, daß der Tracer eine vom zu bestimmenden Wirkstoff abweichende Molekülstruktur aufweist, die nicht durch die Markierung hervorgerufen ist, und eine erheblich höhere oder eine erheblich niedrigere Affinität zum Antikörper hat als der Wirkstoff selbst.

8. Testkit nach Anspruch 7, dadurch gekennzeichnet, daß der zu bestimmende Wirkstoff Thyroxin, Trijodthyronin oder ein Steroid-Hormon ist.

9. Testkit nach Anspruch 7, dadurch gekennzeichnet, daß der unmarkierte Antikörper auf der inneren Oberfläche eines Teströhrchens aufgebracht ist.

**Claims**

1. A method for determining the concentration of the free fraction of an active compound present in a biological fluid, in the presence of natural binders, the free and bound fractions of the active compound being in mutual equilibrium and the quantity of the unlabeled antibody and/or its affinity for the active compound being so small that they do not substantially affect the equilibrium between the free and bound fractions of the active compound, by

   a) contacting a sample of the fluid with an unlabeled antibody,
   b) separating the sample from unlabeled antibodies,
   c) incubating the unlabeled antibody with a labeled substance (tracer) for cross-reaction with the antibody and
   d) measuring the amount of tracer which is or is not bound to the antibody and calculating from this the concentration of the free fraction of the active compound,

   wherein the tracer has a molecular structure which differs from that of the active compound to be determined and which has not been brought about by the labeling, and has an affinity for the antibody which is substantially higher or substantially lower than that of the active compound itself.

2. The method as claimed in claim 1, wherein the free fraction of the active compound to be determined is thyroxine, triiodothyronine or a steroid hormone.

3. The method as claimed in claim 1, wherein the unlabeled antibody is a polyclonal or monoclonal antibody.

4. The method as claimed in claim 1, wherein the tracer is labeled with a radioactive atom, a fluorescing or a chemiluminescent group, an enzyme or a photochromophor.

5. The method as claimed in claim 4, wherein the tracer used for determining the free fraction of thyroxine or triiodothyronine is a thyroxine or triiodothyronine derivative which has been modified at the amino group, at the carboxyl group or at another site in the molecule.

6. The method as claimed in claim 5, wherein the tracer is labeled with radioactive iodine 125.

7. A test kit for determining the concentration of the free fraction of an active compound present in a biological fluid, as claimed in claim 1, wherein the tracer has a molecular structure which differs from that of the active compound to be determined and which has not been brought about by the labeling, and has an affinity for the antibody which is substantially higher or substantially lower than that of the active compound itself.

8. The test kit as claimed in claim 7, wherein the active compound to be determined is thyroxine, triiodothyronine or a steroid hormone.

9. The test kit as claimed in claim 7, wherein the unlabeled antibody has been applied to the inner surface of a small test tube.

**Revendications**

1. Procédé pour déterminer la concentration de la fraction libre d'un agent actif présent dans un liquide biologique, en présence d'agents de liaison naturels, les fractions libre et liée de l'agent actif étant en équilibre et la quantité d'anticorps non marqué et/ou son affinité pour l'agent actif étant si petite(s) qu'elle(s) n'exerce(nt) pas d'influence significative sur l'équilibre entre les fractions libre et liée de l'agent actif, dans lequel:

   a) on met un échantillon du liquide en contact avec un anticorps non marqué,

   b) on sépare l'échantillon des anticorps non marqués,

   c) on incube l'anticorps non marqué avec une substance (traceur) qui réagit de façon croisée avec lui,

   d) on détermine la fraction du traceur qui est liée aux anticorps, ou non liée a ceux-ci, et on en déduit par calcul la concentration de la fraction libre de l'agent actif, caractérisé en ce que le traceur présente une structure moléculaire différente de celle de l'agent actif à déterminer, non consécutive au marquage, et a une affinité pour l'anticorps beaucoup plus élevée ou beaucoup plus petite que l'agent actif lui-même.

2. Procédé selon la revendication 1, caractérisé en ce que la fraction libre de l'agent actif à déterminer est de la thyroxine, de la triiodothyronine ou une hormone stéroïde.

3. Procédé selon la revendication 1, caractérisé en ce que l'anticorps non marqué est un anticorps monoclonal ou polyclonal.

4. Procédé selon la revendication 1, caractérisé en ce qu'on marque le traceur avec un atome radioactif, un groupe fluorescent ou chimiluminescent, une enzyme ou un photochromophore.

5. Procédé selon la revendication 4, caractérisé en ce que, pour doser la fraction libre de thyroxine ou de triiodothyronine, on utilise comme traceur un dérivé de la thyroxine ou de la triiodothyronine qu on a modifié sur le groupe amino, sur le groupe carboxy ou en un autre endroit de la molécule.

6. Procédé selon la revendication 5, caractérisé en ce qu'on effectue le marquage du traceur avec de l'iode-125 radioactif.

7. Coffret d'analyse pour déterminer la concentration de la fraction libre d'un agent actif présent dans un liquide biologique selon la revendication 1, caractérisé en ce que le traceur présente une structure moléculaire différente de celle de l'agent actif à déterminer, non consécutive au marquage, et a une affinité pour l'anticorps beaucoup plus élevée ou beaucoup plus petite que l'agent actif lui-même.

8. Coffret d'analyse selon la revendication 7, caractérisé en ce que l'agent actif à doser est la thyroxine, la triiodothyronine ou une hormone stéroïde.

9. Coffret d'analyse selon la revendication 7, caractérisé en ce qu'on introduit l'anticorps non marqué sur la surface interne d'un tube à essais.

FIG.1

% Bindung

% Bindung

FIG.2

Antikörper: 20 nl / tube

+——+ 50 µl Standard

o——o 100 µl Standard

•——• 200 µl Standard

pg FT 4 / ml

pg FT 4 / ml

EP 0 257 352 B1

EP 0 257 352 B1

# FIG.3

**% Bindung**

Antikörper: 80 n l /tube

+———+   50 µl   Standard
o———o   100 µl   Standard
•———•   200 µl   Standard

pg  FT 4 /ml

# FIG.4

**% Bindung**

+———+   Tracer: 0.04 µCi / ml
o———o   Tracer: 0.02 µCi / ml

pg  FT 4 /ml

FIG. 6

FIG. 5

EP 0 257 352 B1

% Bindung

FIG.7

100

80

60

40

20

0

0  50  100  150  200

⟶ pg FT 4/ml

% Bindung

FIG.8

100

80

60

40

20

0

0  50  100  150  200

⟶ pg FT 4/ml